Europäisches Patentamt

(19) **European Patent Office** (11) Numéro de publication: **0 032 138**

Office européen des brevets **B1**

(12) **FASCICULE DE BREVET EUROPEEN**

(45) Date de publication du fascicule du brevet: **21.11.84** (51) Int. Cl.³: **A 61 F 5/44**

(21) Numéro de dépôt: **81400008.9**

(22) Date de dépôt: **06.01.81**

(54) **Dispositif pour recueillir les urines d'incontinents masculins.**

(30) Priorité: **07.01.80 CH 75/80**

(43) Date de publication de la demande:
**15.07.81 Bulletin 81/28**

(45) Mention de la délivrance du brevet:
**21.11.84 Bulletin 84/47**

(84) Etats contractants désignés:
**AT BE DE FR GB IT LU NL SE**

(56) Documents cités:
**DE-C- 674 158**
**FR-A- 527 610**
**FR-A-2 187 277**
**GB-A-1 274 374**
**US-A-3 526 227**

(73) Titulaire: **LABORATOIRES BIOTROL S.A.**
**1, rue du Foin**
**F-75140 Paris Cedex 03 (FR)**

(72) Inventeur: **Ozenne, Jean-Pierre**
**6, rue de l'Orée du Bois**
**F-60580 Coye-La-Foret (FR)**

(74) Mandataire: **Phélip, Bruno et al**
**c/o Cabinet Harlé & Phélip 21, rue de la**
**Rochefoucauld**
**F-75009 Paris (FR)**

Courier Press, Leamington Spa, England.

EP 0 032 138 B1

# Description

La présente invention concerne un dispositif destiné à recueillir les urines chez les hommes, notamment chez les hommes souffrant d'incontinence.

L'incontinence urinaire affecte une population non négligeable de patients.

Elle peut être d'origine congénitale, mais survient le plus souvent à la suite d'un accident corporel ou d'une intervention chirurgicale; en particulier, une ablation de la prostate est fréquemment suivie d'une inaptitude à la rétention urinaire.

D'autre part, la situation des individus devant rester alités, ou grabataires, implique qu'on recueille leurs urines.

On a déjà proposé un grand nombre de solutions pour la collecte des urines de patients de sexe masculin souffrant d'incontinence urinaire ou ne pouvant se lever. Ces solutions peuvent se classer en trois catégories:

1. Dans le cas de patients atteints d'une incontinence partielle, ceux-ci utilisent seulement des couches absorbantes, telles que celles décrites par exemple dans le brevet FR—1 468 946.

Le maintien en place de ces couches est assuré par des culottes de divers types.

Mais ces couches, outre le fait qu'elles ne peuvent rendre service qu'à une infime minorité d'incontinents, présentent le défaut de provoquer rapidement des irritations dues au contact de l'épiderme avec les tissus imprégnés d'urine.

2. Dans les cas d'incontinence les plus sévères, c'est-à-dire quand les émissions d'urine peuvent être brutales et importantes, il est préconisé de collecter le liquide dans un sac vidangeable. Cela peut se faire au moyen d'étuis péniens, prolongés par un tube d'écoulement dont le but est de conduire l'urine jusqu'à un sac réservoir (voir en particulier les brevets FR—1 498 634, 1 508 356, 1 523 589, 1 591 685, 2076932, 2083122 et 2364645).

Ce type d'appareil est essentiellement constitué d'un condom en matière souple, notamment en latex, qui vient enserrer le pénis. La fixation d'un tel étui sur la verge est difficile et la comprime nécessairement.

Dans certaines de leurs variantes, ces appareils impliquent même l'utilisation de produits adhésifs destinés au collage de l'étui sur la verge, et provoquent donc des irritations et des strictions de celle-ci.

3. Plus particulièrement pour des hommes actifs, il est déjà apparu dans le passé préférable de réaliser un dispositif collecteur d'urine constitué d'un ensemble de plusieurs éléments dont la structure et l'agencement permettent une plus grande autonomie et un meilleur confort. Ce type d'appareillage se distingue des précédents en ce qu'il comporte, de manière générale, une partie fixée au corps, qui fait office de réceptacle et assure le joint avec la verge, et à laquelle est reliée une poche de recueil, généralement vidangeable. Ainsi dans les brevets DE—A—674 158 et FR—A—527 610 on décrit des poches de recueil munies d'une ouverture latérale circulaire sur le pourtour de laquelle est fixé à demeure un anneau de raccordement venant s'adapter à un dispositif collecteur situé sur le patient.

On trouve aussi décrit dans le brevet FR—1 219 897 un tel système comprenant une poche de recueil renfermant un matériau spongieux destiné à pomper les urines.

Par ailleurs, dans le brevet FR—A—2242964, on décrit un système simple rentrant également dans cette catégorie et comportant un diaphragme élastique destiné à enserrer la verge de façon étroite et une couche de matière collante pour maintenir la poche de recueil par adhérence sur la face extérieure du système.

On connaît également un dispositif pour le recueil des urines d'incontinents masculins, actuellement mis sur le marché sous la dénomination commerciale "Disposan", par la Société AB Medettprodukter, et qui peut être décrit comme suit: il comporte une ceinture avec lanières, qui permet de maintenir dans la région pubienne une bande de tissu comportant une perforation circulaire déformable au travers de laquelle passe la verge, ainsi qu'un premier anneau, rigide et muni d'une gorge radiale apte à être maintenue par la bande de tissu après engagement d'une face de cet anneau dans la perforation circulaire susdite; un manchon en latex en forme de tronc de cône de révolution vient se fixer élastiquement par sa base de plus grand diamètre sur le rebord du premier anneau le plus proche de la région pubienne et pénètre dans l'alésage de l'anneau, le rebord correspondant au plus petit diamètre de cette pièce tronconique venant alors enserrer la verge et assurer un joint étanche à ce niveau. Pour compléter ce dispositif, une poche de recueil, destinée à être montée sur un second anneau, semi-rigide, indépendant, est encore nécessaire. Cette poche a globalement la forme d'un étui, composé de deux films plastiques soudés entre eux et dans lequel est prévu un système anti-reflux, également constitué par deux films plastiques, de longueur inférieure aux précédents, soudés entre eux et au bord ouvert de la poche, de manière à définir une goulotte dont l'extrémité inférieure est, en service, assez faible pour assurer l'absence de reflux.

En pratique, ce dispositif est encore complété par un tube d'écoulement situé à l'extrémité inférieure de la poche (en service) et qui permet de faire déverser la poche dans un réservoir complémentaire, si nécessaire.

Le montage d'un tel dispositif implique que l'utilisateur doive retrousser l'extrémité libre de la poche sur le second anneau, selon une configuration inverse de celle décrite dans le brevet FR—1 365 704, et ensuite emmancher le tout, à force, sur le rebord libre de la gorge radiale du premier anneau, tandis que le manchon élastique tronconique a au préalable été inséré dans celui-ci et fixé sur son rebord côté pubis.

Lorsqu'il est encliqueté sur le premier anneau, ce second anneau presse le film de la poche sur la partie extérieure du rebord du premier anneau, sur lequel l'encliquetage se fait.

Les principaux inconvénients de ce système résident dans la difficulté de mise en place de la poche de recueil sur le second anneau et dans le manque d'étanchéité du joint entre la poche et le premier anneau, à cause de la formation inévitable de plis sur le film de la poche et des lignes de soudure latérales de la poche.

Les patients sont alors conduits, pour tenter de remédier un tant soit peu à cet inconvénient, à enduire le film de la poche avec des produits visqueux, tels que des graisses, destinés à améliorer l'étanchéité du joint.

D'autre part, la poche ainsi fixée débouche axialement sur le second anneau et, une fois le dispositif mis en place sur le patient, elle se trouve nécessairement pliée à 90° directement au ras du second anneau, et les plis formés à ce niveau sont très inconfortables pour l'utilisateur, dont la verge est en contact avec eux.

Il était donc souhaitable, pour améliorer le confort des porteurs de poches de recueil d'incontinence urinaire, de concevoir un appareil se distinguant de ceux-ci.

L'objet de la présente invention est un dispositif qui satisfait à cet objectif et qui, simultanément, réalise le recueil des urines d'incontinents dans des conditions parfaites ou quasi-parfaites d'étanchéité, tout en étant d'une configuration simplifiée et d'une mise en place particulièrement aisée, sans que la poche se trouve lors de son utilisation pliée à 90°C au ras du raccord d'entrée.

L'invention a pour objet un dispositif de recueil des urines d'incontinents masculins, comportant essentiellement une ceinture munie d'une pièce de matière déformable comportant une perforation circulaire, un premier anneau rigide, comprenant une gorge radiale apte à être retenue par la pièce de matière déformable après engagement dans la perforation circulaire de celle-ci, un manchon élastique tronconique ayant son extrémité de plus grand diamètre appropriée pour être retroussée et plaquée élastiquement sur la paroi côté pubis du premier anneau, et un second anneau, semirigide, comportant une collerette munie d'un bourrelet terminal aptes à le rendre encliquetable sur la premier anneau, et une poche de recueil, tandis que la perforation circulaire, le premier anneau et le second anneau doivent permettre le libre passage de la verge dans leurs alésages, ce dispositif étant caractérisé en ce que la poche de recueil est composée de deux parois souples juxtaposées soudées entre elles sur la totalité de leurs bords périphériques, à l'exception éventuellement d'une partie étroite dans sa zone inférieure pour permettre un écoulement contrôlé du liquide, en ce que l'ouverture d'entrée de ladite poche est ménagée dans la partie supérieure de l'une des parois et en ce que le bord plan de ladite ouverture est accolé à demeure et de manière étanche à une face plane radiale du second anneau.

Selon une variante avantageuse de ce dispositif, la fixation, notamment par soudure ou collage, du second anneau sur le pourtour de l'ouverture latérale circulaire de la poche de recueil est réalisée selon une couronne circulaire dont le diamètre est inférieur à celui de la gorge que définissent dans ce second anneau la collerette et le bourrelet terminal de celle-ci. Dans ce dispositif, en effet, toute traction volontaire ou non, excercée sur la poche de recueil a pour conséquence, non pas une désolidarisation de celle-ci du reste du dispositif, mais au contraire, au moins dans un premier temps, un effort accru de serrage de la collerette du second anneau sur le premier anneau sur lequel celui-ci est encliqueté, sous l'effet d'un rétrécissement du diamètre de l'extrémité de la collerette du second anneau comportant le bourrelet susdit. Il faut alors un effort de traction nettement plus important, ou réparti en au moins deux points suffisamment éloignés l'un de l'autre, pour qu'on parvienne à l'effet inverse, qui consiste en un arrachement de la poche et du second anneau assemblés et résulte d'une déformation allant cette fois-ci dans le sens d'un élargissement du diamètre du bourrelet porté par la collerette du second anneau.

Selon une autre variante, plus particulièrement avantageuse, du dispositif selon l'invention, celui-ci comprend un premier anneau, rigide et comprenant une gorge, dont la paroi côté poche comporte un épaulement externe ayant un diamètre extérieur correspondant sensiblement au diamètre de l'alésage du second anneau. De préférence, le diamètre de l'alésage du premier anneau, épaulement compris, est variable et tel qu'il passe par un minimum (éventuellement maintenu constant sur une certaine fraction de l'épaisseur de l'anneau) vers le milieu de l'épaisseur de cet anneau, et croît à mesure qu'on se rapproche de chacun des bords respectifs de celui-ci, avantageusement de façon à définir en section droite une courbe ou deux fractions de courbe, dans laquelle le profil intérieur du second anneau s'intègre sans faire de saillie dans la zone de passage de la verge et ayant pour effet d'améliorer le confort de l'utilisateur en évitant les arêtes vives ou autres discontinuités génératrices de frottements irritants.

En pratique, le dispositif selon l'invention est réalisé-hormis la ceinture à lanières qui peut être en matière textile, naturelle ou synthétique- en des matières plastiques ou caoutchouteuses diverses, dont les types et qualités sont appropriés pour chacun des éléments du dispositif. Le choix des matières plastiques et des spécifications les plus appropriées pour celles-ci est à la portée de l'homme de l'art. En particulier, l'anneau rigide est avantageusement en polyamide, tandis que l'anneau semi-rigide est avantageusement réalisé en matière thermoplastique, telle qu'une polyoléfine, du PVC, un polyuréthane ou autres.

D'autre part, les deux parois de la gorge du premier anneau peuvent n'être pas semblables et, outre le fait que la paroi sur laquelle est encliqueté le second anneau peut porter un épaulement comme on l'a indiqué plus haut, la paroi opposée à celle-ci peut avoir un diamètre externe différent de celui de la précédente, et de préférence supérieur à lui.

L'invention est illustrée plus concrètement en référence aux dessins annexés, qui complètent la présente description en montrant un exemple de réalisation du dispositif selon l'invention, ne limitant pas celle-ci, et dans lesquels:

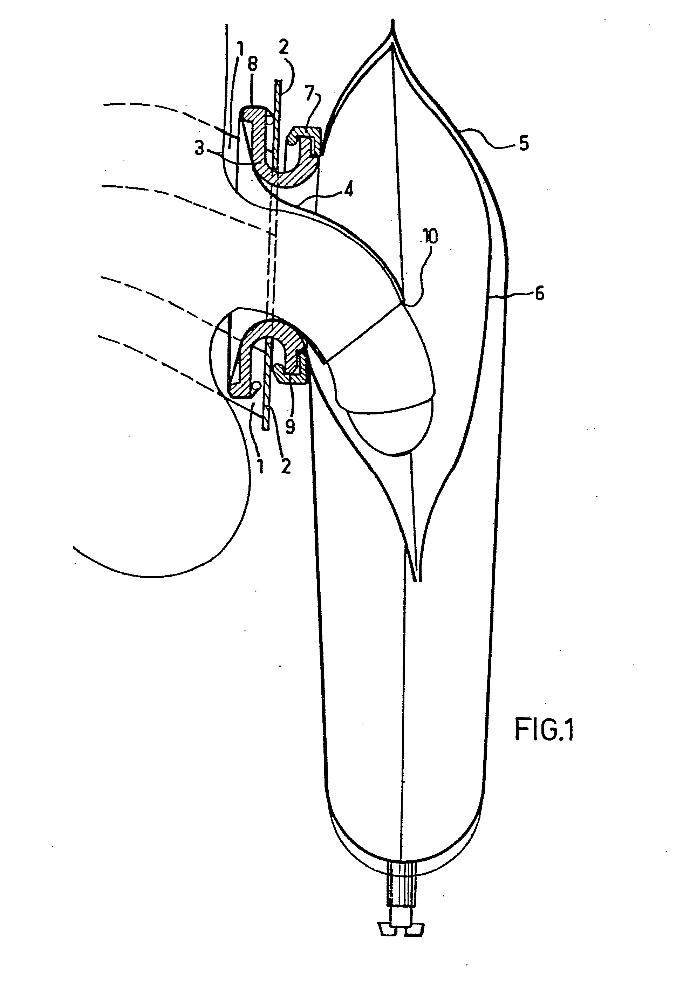

Fig. 1 est une vue schématique en coupe du dispositif porté par l'utilisateur, à l'exception de la ceinture, qui a été omise pour ne pas surcharger inutilement le schéma;

Fig. 2 est une vue en coupe transversale d'une variante avantageuse du second anneau de ce dispositif, et

Fig. 3 est une vue en coupe transversale du troisième anneau et d'une partie de la poche de recueil montrant un mode de fixation tout particulièrement avantageux de la poche sur cet anneau.

Le dispositif représenté, utile pour recueillir les urines d'incontinents urinaires masculins, comporte, outre la ceinture et la pièce de matière déformable avec perforation circulaire 2, un premier anneau en matière plastique rigide 3, comportant une gorge radiale apte à être retenue par la pièce de matière déformable après engagement dans la perforation circulaire de celle-ci, un manchon tronconique en latex 4, dont l'extrémité de plus grand diamètre (avantageusement munie d'un bourrelet terminal) est retroussée et plaquée élastiquement sur le plus grand diamètre de paroi du premier anneau comportant deux parois définissant une gorge externe, ainsi qu'une poche de recueil en matière plastique souple 5, avantageusement pourvue d'une poche interne anti-reflux 6, et d'un second anneau semi-rigide 7, solidaire de cette poche et apte à s'encliqueter sur la seconde paroi du premier anneau 3.

L'étanchéité du système est assurée en trois points différents:

. en 8, où le grand diamètre du manchon tronconique en latex est retroussé sur une des parois de gorge du premier anneau;
. en 9, où se fait l'encliquetage en force du second anneau en matière plastique semi-rigide sur le premier anneau en matière plastique rigide;
. en 10, entre le latex et la verge.

On notera que le problème d'étanchéité entre le second anneau et la poche n'existe plus dans ce dispositif.

Ce sont là des avantages propres au dispositif selon l'invention et qui le rendent nettement plus performant que ceux que l'on connaissait jusqu'alors.

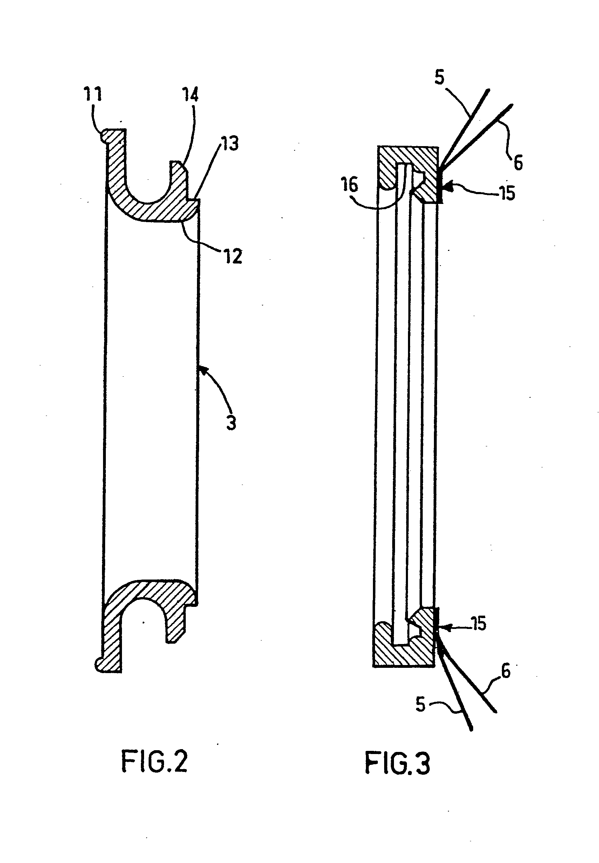

Le premier anneau comprend, dans la variante préférée qui est illustrée en coupe sur la figure 2, un bourrelet extérieur 11 sur l'une des parois de la gorge, un alésage profilé 12, un épaulement 13 et, si on le souhaite, également un chanfreinage 14 destiné à permettre un encliquetage facile du second anneau et assurer un joint plus efficace encore entre celui-ci et le premier anneau.

Le bourrelet 11 a pour fonction d'améliorer encore l'étanchéité du joint avec le manchon tronconique en latex tendu autour de lui.

Si l'on se réfère à la figure 3, on voit que la variante avantageuse illustrée, en ce qui concerne le second anneau, consiste en un anneau ayant sensiblement le profil indiqué sur cette figure, sur lequel la poche 5 est fixée, par soudure, selon une couronne circulaire 15 dont le diamètre est inférieur à celui de la gorge intérieure 16 du second anneau.

Outre les avantages déjà mentionnés, le dispositif conforme à l'invention, ou tout dispositif reprenant les éléments essentiels de celui-ci et qui en serait donc un strict équivalent, ne provoque pas d'irritations au patient qui le porte, ne nécessite aucun produit supplémentaire, en particulier du fait que tous les joints ont dans ce dispositif une étanchéité suffisante, et permet en outre, par une simple rotation du second anneau par rapport au premier, de faire varier à convenance l'orientation de la poche de recueil.

**Revendications**

1. Dispositif de recueil des urines d'incontinents masculins, comportant essentiellement une ceinture (1) munie d'une pièce de matière déformable (2) comportant une perforation circulaire, un premier anneau (3), rigide, comprenant une gorge radiale apte à être retenue par la pièce de matière déformable après engagement dans la perforation circulaire de celle-ci, un manchon élastique tronconique (4) ayant son extrémité de plus grand diamètre appropriée pour être retroussée et plaquée élastiquement sur la paroi côté pubis du premier anneau, un second anneau (7), semi-rigide, comportant une collerette munie d'un bourrelet terminal aptes à le rendre encliquetable sur le premier anneau, et une poche de recueil (5), tandis que la perforation circulaire, le premier anneau et le second anneau doivent permettre le libre passage de la verge dans leurs alésages, caractérisé en ce que la poche de recueil (5) est composée de deux parois souples juxtaposées soudées entre elles sur la totalité de leurs bords périphériques, à l'exception éventuellement d'une partie étroite dans sa zone inférieure pour permettre un écoulement contrôlé du liquide en ce que l'ouverture d'entrée de ladite poche est ménagée dans la partie supérieure de l'une des parois et en ce que le bord plan de ladite ouverture est accolé à demeure et de manière étanche à une face plane radiale du second anneau (7).

2. Dispositif selon la revendication 1, caractérisé en ce que la fixation du second anneau (7) sur le pourtour de l'ouverture latérale circulaire de la poche de recueil (5) est réalisée selon une couronne circulaire (15) dont le diamètre est inférieur à celui de la gorge (16) que définissent dans ce second anneau la collerette et le bourrelet terminal de celle-ci.

3. Dispositif selon l'une des revendications 1 ou 2, caractérisé en ce qu'il comprend un premier anneau (3), rigide et comprenant une gorge, dont la paroi côté poche comporte un épaulement externe (13) ayant un diamètre extérieur correspondant sensiblement au diamètre de l'alésage du second anneau.

4. Dispositif selon la revendication 3, caractérisé en ce que le diamètre (12) de l'alésage du premier anneau (3), épaulement (13) compris, est variable et tel qu'il passe vers le milieu de l'épaisseur de cet anneau par un minimum, éventuellement maintenu constant sur une certaine fraction de l'épaisseur de l'anneau, et croît à mesure qu'on se rapproche de chacun des bords respectifs de celui-ci, de façon à définir en section droite une courbe ou deux fractions de courbe, dans laquelle le profil intérieur du second anneau s'intègre sans faire de saillie dans la zone de passage de la verge.

**Patentansprüche**

1. Urinsammelvorrichtung für Männer mit Inkontinenz, umfassend im wesentlichen einen Gürtel (1), versehen mit einem einen runden Durchbruch aufweisenden Teil (2) aus deformierbarem Material, einem ersten starren Ring (3) mit einer Radialnut, die von dem Teil aus deformierbarem Material nach Einfügen in dessen runden Durchbruch festhaltbar ist, eine elastische kegelstumpfförmige Manschette (4), deren Ende größeren Durchmessers ausgebildet ist, um umgestülpt und auf der schamseitigen Wandung des ersten Ringes elastisch angeschlagen zu werden, einen zweiten, halbstarren Ring (7) mit einem Kragen, der mit einem zum Einklinken auf dem ersten Ring ausgebildeten Endwulst versehen ist, und eine Auffangtasche (5), während der runde Durchbruch, der erste Ring und der zweite Ring den freien Durchtritt des Penis in ihre Öffnungen zulassen müssen, dadurch gekennzeichnet, daß die Auffangtasche (5) aus zwei weichen übereinanderliegenden, längs der Gesamtheit ihrer Umfangskanten miteinander verschweißten Wandungen besteht, gegebenenfalls mit Ausnahme eines engen Bereichs in ihrer unteren Zone zum Ermöglichen des gesteuerten Ablassens der Flüssigkeit, daß die Einlaßöffnung der genannten Tasche in den oberen Abschnitt einer der Wandungen eingearbeitet ist und daß die ebene Kante der genannten Öffnung dauerhaft und abdichtend an einer ebenen radialen Seite des zweiten Rings (7) festgelegt ist.

2. Vorrichtung nach Anspruch 1, dadurch gekennzeichnet, daß die Befestigung des zweiten Ringes (7) auf dem Umfang der seitlichen runden Öffnung der Auffangtasche (5) längs einer Krone (15) vorgenommen ist, deren Durchmesser kleiner ist als der der Nut (16), welche in diesem zweiten Ring den Kragen und den Endwulst derselben definiert.

3. Vorrichtung nach einem der Ansprüche 1 oder 2, dadurch gekennzeichnet, daß sie einen ersten starren Ring (3) mit einer Nut umfaßt, deren taschenseitige Wandung eine Außenschulter (13) mit einem Außendurchmesser umfaßt, der im wesentlichen dem Öffnungsdurchmesser des zweiten Ringes entspricht.

4. Vorrichtung nach Anspruch 3, dadurch gekennzeichnet, daß der Durchmesser (12) der Öffnung des ersten Ringes (3) einschließlich der Schulter (13) variabel und derart ist, daß er in Richtung der Mitte der Ringdicke durch ein Minimum geht und zunimmt, je mehr man sich jeweils einer der Kanten derselben nähert, derart, daß im Querschnitt eine Kurve oder zwei Kurvenabschnitte definiert werden, in die das Innenprofil des zweiten Ringes sich einfügt, ohne in die Zone des Penisdurchtritts zu ragen.

**Claims**

1. Device for collecting urine from male incontinents, essentially incorporating a belt (1) fitted with a piece of a deformable substance (2) incorporating a circular perforation, a first ring (3) which is rigid, comprising a radial groove capable of being retained by the piece of deformable substance after being engaged in the circular perforation in the latter, a frusto-conical elastic sleeve (4) having its wider-diameter end suitable for being rolled up and laid elastically over the pubis-side wall of the first ring, a second, semi-rigid ring (7), incorporating a collar equipped with an end rim suitable to make it capable of being snapped onto the first ring and a collecting pouch (5), while the circular perforation, the first ring and the second ring must permit free passage of the penis in their bores, characterised in that the collecting pouch (5) consists of two flexible walls placed side by side and welded together over the whole of their peripheral edges, with the possible exception of a narrow part in its lower region to permit a controlled flow of the liquid, in that the entry opening of the said pouch is arranged in the upper part of one of the walls, and in that the planar edge of the said opening is joined permanently and in a leaktight manner to a planar radial face of the second ring (7).

2. Device according to Claim 1, characterised in that the fixing of the second ring (7) on the periphery of the circular side opening of the collecting pouch (5) is made according to a circular crown-ring (15) whose diameter is smaller than that of the groove (16) which is defined in this second ring by the collar and the end rim of the latter.

3. Device according to either of Claims 1 or 2, characterised in that it comprises a first, rigid, ring (3) comprising a groove whose pouch-side wall incorporates an outer shoulder (13) having an outer diameter substantially corresponding to the diameter of the bore of the second ring.

4. Device according to Claim 3, characterised in that the diameter (12) of the bore of the first ring (3) including the shoulder (13), is variable and such that it passes towards the middle of the thickness of this ring through a minimum, maintained constant, if appropriate, over some fraction of the thickness of the ring, and increases on approaching each of the respective edges of the latter, so as to define in a straight cross-section a curve, or two portions of a curve, which the inner outline of the second ring joins without forming a projection in the region for the passage of the penis.

FIG.1

FIG.2

FIG.3